(19) Europäisches Patentamt
European Patent Office
Office européen des brevets

(11) Veröffentlichungsnummer: **0 128 443**
**B1**

(12) # EUROPÄISCHE PATENTSCHRIFT

(45) Veröffentlichungstag der Patentschrift: **14.01.87**

(51) Int. Cl.⁴: **C 08 G 16/02**

(21) Anmeldenummer: **84106043.7**

(22) Anmeldetag: **28.05.84**

(54) **Verfahren zur Herstellung von Poly-alpha-cyanacrylsäureestern und deren Depolymerisation.**

(30) Priorität: **09.06.83 DE 3320796**

(43) Veröffentlichungstag der Anmeldung: **19.12.84 Patentblatt 84/51**

(45) Bekanntmachung des Hinweises auf die Patenterteilung: **14.01.87 Patentblatt 87/3**

(84) Benannte Vertragsstaaten: **AT BE CH DE FR GB IT LI NL SE**

(56) Entgegenhaltungen:
**DE-A-2 104 518**
**GB-A-1 158 997**
**US-A-2 467 927**
**US-A-2 721 858**

(73) Patentinhaber: **BAYER AG, Konzernverwaltung RP Patentabteilung, D-5090 Leverkusen 1 Bayerwerk (DE)**

(72) Erfinder: **Waniczek, Helmut, Dr., Wolfskaul 2, D-5000 Koeln 80 (DE)**
Erfinder: **Bartl, Herbert, Dr., Eichendorffweg 10, D-5068 Odenthal (DE)**

**EP 0 128 443 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein neues Verfahren zur Herstellung von Poly-α-cyanacrylsäureestern und deren Depolymerisation.

Poly-α-cyanacrylsäureester stellen wichtige Ausgangsprodukte für die Synthese von monomeren α-Cyanacrylsäureestern dar. Diese können als hochwertige Klebstoffe Verwendung finden. Die monomeren α-Cyanacrylsäureester werden z.B. durch thermisch-katalytische Depolymerisation von Polycyanacrylat hergestellt. Ausbeute, Reinheit, Stabilität und Abbindegeschwindigkeit hängen von der Art der Herstellung des Polycyanacrylates ab.

Die Poly-α-cyanacrylate lassen sich vorteilhaft durch eine Kondensationsreaktion von Formaldehyd- oder Formaldehyd abspaltenden Verbindungen - mit Cyanessigsäureestern unter basischer oder saurer Katalyse herstellen. Dabei muß das entstehende Reaktionswasser entfernt werden, da es einerseits die Estergruppen verseifen kann und andererseits die Depolymerisation behindern kann.

Die bekannten Verfahren sind aufwendig. So werden Lösungsmittel wie Methanol oder Toluol zugesetzt, mit denen zusammen das Wasser destillativ abgetrennt wird (z.B. US-PS 2 721 858, 2 763 677), oder es werden Substanzen zugesetzt, die das Reaktionswasser chemisch binden, z.B. Essigsäureanhydrid oder Maleinsäureanhydrid (z.B. US-PS 2 912 454). Diese Verfahren sind darüber hinaus zeit- und energieaufwendig, da erst die Reaktionswärme abgeführt werden muß, lange Reaktionszeiten nötig sind und der anschließende Destillationsprozeß ebenfalls Zeit und Energie erfordert. Durch die thermische Belastung bei der Destillation wird hergestelltes Polymerisat z.B. durch Verseifungsreaktion geschädigt und die erzielbare Ausbeute vermindert.

Nach den bekannten Verfahren ist es nicht möglich, Polycyanacrylsäureester in größerem Maßstab ohne die Mitverwendung größerer Lösungsmittelmengen herzustellen, da die Reaktionswärme schwer abgeführt werden kann. Andererseits kann die Reaktion einen explosiven Verlauf nehmen, wenn die Reaktionspartner zu rasch vereinigt werden. Vereinigt man die Reaktionspartner genügend langsam, so daß die Reaktionstemperatur nicht übermäßig ansteigt, so kann das Reaktionswasser in vertretbarer Zeit nicht abgetrennt werden, ohne das Polymerisat z.B. durch Verseifungsreaktionen zu schädigen.

Es wurde nun gefunden, daß diese Nachteile vermieden werden können, wenn man in kontinuierlicher Verfahrensweise das Polymerisat durch Reaktion von Cyanessigestern mit Formaldehyd oder Formaldehyd abspaltenden Substanzen bei Temperaturen von 50 - 200°C, vorzugsweise 80 - 120°C in Gegenwart von Katalysatoren herstellt. Die Verweilzeit der Reaktionskomponenten in der Reaktionszone beträgt weniger als 20 Minuten und das Reaktionswasser kann im Vakuum kontinuierlich abdestilliert werden.

Gegenstand der Erfindung ist daher ein Verfahren zur Herstellung von Poly- α -cyanacrylsäureestern durch Umsetzung von Formaldehyd oder Formaldehyd abspaltenden Verbindungen mit Cyanessigsäureestern in Gegenwart basischer Katalysatoren, dadurch gekennzeichnet, daß die Umsetzung kontinuierlich bei Temperaturen von 50 - 200°C, vorzugsweise von 80 - 150°C, bei einem Druck von 1 bis 20 bar und einer Verweilzeit von weniger als 20 Minuten durchgeführt wird, indem man das Reaktionsgemisch in einen Reaktor kontinuierlich einspeist, mit dem Katalysator vermischt, das Gemisch bei Reaktionstemperatur durch eine Reaktionszone führt, das Reaktionswasser kontinuierlich und gegebenenfalls bei vermindertem Druck abdestilliert.

Besonders geeignet für dieses Verfahren sind spezielle Reaktionsschneckenmaschinen. Diese sind in verschiedene Zonen eingeteilt:

1. Dosierzone für Paraformaldehyd und Cyanessigsäureestern
2. Aufheizzone
3. Dosierzone für Katalysatoren
4. Polymerisationszone
5. Entgasungszone
6. Extrudierzone

Figur 1 zeigt beispielhaft den schematischen Aufbau einer derartigen Vorrichtung.

Nach der Entgasungszone können sich weitere Polymerisations- und Entgasungszonen anschließen.

Das erfindungsgemäße Verfahren zur Herstellung von Polycyanacrylat wird so durchgeführt, daß die Reaktionskomponenten, z.B. Paraformaldehyd und Cyanessigsäuremethylester im Molverhältnis 1 : 2 bis 2 : 1, bevorzugt 1 : 1,5 bis 1,5 : 1 getrennt oder gemeinsam in die Einzugszone (1) des Extruders dosiert werden, dort auf die Reaktionstemperatur erwärmt und in der Dosierzone (3) mit dem Katalysator vereinigt werden. Nach der Polymerisationszone (4) erhält man eine viskose Masse, die im Vakuum entgast wird (5) (z.B. Entfernung von unter den dort herrschenden Bedingungen flüchtigen Verbindungen wie Wasserdampf, flüchtigen Katalysatoren, flüchtigen Reaktionsprodukten). Dieser Vorgang kann wiederholt werden. In der Extrudierzone (6) wird ein viskoses, farbloses, entgastes Polycyanacrylat als Schmelze erhalten, das direkt oder gegebenenfalls nach Zugabe von üblichen Stabilisatoren oder weiteren üblichen Verarbeitungshilfsmitteln weiter verarbeitet werden kann.

Als Extruder kann zum Beispiel eine Zweiwellenschneckenpresse verwendet werden. Die Entgasungszonen sollen eine ausreichende Länge zur destillativen Abtrennung der flüchtigen Reaktionsprodukte besitzen, wobei der Druck in der ersten Entgasungszone höher sein kann als in der zweiten. Als günstig hat sich in der ersten Entgasungszone ein Druck von 500 - 700 mbar und in der zweiten ein Druck von 10 - 5 mbar erwiesen.

Unter Formaldehyd oder Formaldehyd abspaltenden Substanzen ist Formaldehyd als Gas oder kondensierte

Phase, dessen Lösungen in Wasser, organischen Lösungsmitteln oder Gemischen derselben zu verstehen. Weiterhin können Substanzen verwendet werden, die unter den Reaktionsbedingungen Formaldehyd abspalten, z.B. Polymere des Formaldehyds wie Paraldehyd (Polyoxymethylen) oder Umsetzungsprodukte von Formaldehyd mit Alkoholen wie Halbacetalen, z.B. Monomethylformal oder Monoethylformal.

Erfindungsgemäß können Cyanessigsäureester der Formel

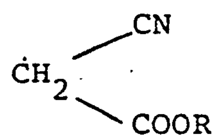

,

in welcher

R einen Alkyl-, Halogenalkyl-, Alkoxyalkyl-, Alkenyl-, Cycloalkyl- oder Phenylrest mit jeweils 1 -10 C-Atomen bedeutet

eingesetzt werden.

Im erfindungsgemäßen Verfahren können Katalysatoren eingesetzt werden in reiner Form, in Form verdünnter Lösungen in Wasser, organischen Lösungsmitteln oder Cyanessigestern eingesetzt werden.

Es werden basische Verbindungen als Katalysatoren eingesetzt, z.B. anorganische Basen wie NH, Alkalihydroxide wie NaOH und KOH, organische Basen wie primäre, sekundäre oder tertiäre Alkyl-, Aryl- oder Cycloalkylamine wie Butylamin, Triethylamin oder Polyethylamin, cyclische Amine wie Piperidin, Morpholin oder Pyridin und deren Derivate. Bevorzugt werden organische Amine, besonders bevorzugt Piperidin.

Pro Mol Cyanessigsäureester werden 0,001 bis 0,05 mol, bevorzugt 0,005 bis 0,03 mol Katalysator eingesetzt.

Wenn als Katalysator flüchtige Verbindungen eingesetzt werden, so wird in der Entgasungsstufe gemeinsam mit den flüchtigen Reaktionskomponenten auch der Katalysator ganz oder teilweise aus der Polycyanacrylatschmelze abgetrennt.

Das Reaktionsgemisch, bestehend aus dem Cyanessigsäureester und Formaldehyd oder Formaldehyd abspaltenden Verbindungen wird mit dem basischen Katalysator kontinuierlich vereinigt und in einer Zone kontinuierlicher Durchmischung bei Temperaturen von 50°C bis 200°C, bevorzugt von 70°C bis 180°C, besonders bevorzugt von 90°C bis 150°C, zur Reaktion gebracht. Der Druck des Reaktionsgemisches beträgt 1 bis 100 bar. Die mittlere Verweilzeit in dieser Reaktionszone beträgt bis zu 20 Minuten, wobei eine mittlere Verweilzeit von 0,5 Minuten bis 15 Minuten bevorzugt und eine mittlere Verweilzeit von 1 Minute bis 10 Minuten ganz besonders bevorzugt wird.

Durch das erfindungsgemäße Verfahren zur Herstellung von Poly-α-cyanacrylat werden sehr reine Polycyanacrylate hergestellt, die bei der Depolymerisation Cyanacrylat in hohen Ausbeuten und Reinheitsgraden liefern. Darüberhinaus ist das erfindungsgemäße Verfahren wirtschaftlich und technisch einfach durchzuführen.

Die nach dem erfindungsgemäßen Verfahren hergestellten Polycyanacrylsäureester können nach bekannten Verfahren kontinuierlich oder diskontinuierlich unter Zusatz von Polymerisationsinhibitoren wie Phosphor(V)oxid, Polyphosphorsäuren, Hydrochinon, Pikrinsäure, Schwefeldioxid, Stickstoffoxide, $CO_2$, Fluorwasserstoff und Sultonen in Gegenwart oder Abwesenheit hochsiedender inerter Flüssigkeiten bei erhöhter Temperatur und im Vakuum zu monomeren Cyanacrylsäureestern depolymerisiert werden (z.B. in US-P 2 763 677, US-P 2 912 454, US-P 2 467 927, DAS 21 04 518 oder DAS 20 27 502).

Die erfindungsgemäß hergestellten Polycyanacrylsaureester können isoliert, oder direkt der Depolymerisation unterzogen werden. Besonders vorteilhaft kann das erfindungsgemäße Verfahren eingesetzt werden, wenn die Depolymerisation zu monomeren Cyanacrylsäureestern kontinuierlich durchgeführt wird, so daß man in einem Verfahrensschritt von den Ausgangsverbindungen direkt zu monomeren Cyanacrylsäureestern gelangt. Dabei kann die Depolymerisation in der gleichen Vorrichtung durchgeführt werden, in der erfindungsgemäß das Polycyanacrylat hergestellt wird, indem nach der Entgasungsstufe der Polycyanacrylatschmelze Polymerisationsinhibitoren und gegebenenfalls hochsiedende Flüssigkeiten zugesetzt werden und in einer weiteren Entgasungszone bei erhöhter Temperatur und bei vermindertem Druck der monomere Cyanacrylsäureester abdestilliert wird.

Das Polymere kann auch vorzugsweise als Schmelze kontinuierlich aus dem Reaktor ausgetragen werden und seiner weiteren Verwendung, z.B. einer Depolymerisation in einer Reaktionsschneckenmaschine, zugeführt werden.

Für die thermisch-katalytische Depolymerisation wurden neben diskontinuierlichen auch kontinuierliche Verfahren z.B. in der DE-AS 2 027 502 oder DE-AS 2 104 518 vorgeschlagen, die aber den Nachteil haben, daß die Ausgangsverbindung, die Poly-α-cyanacrylsäureester diskontinuierlich hergestellt und aufwendig von

Katalysatorresten und Reaktionswasser befreit werden müssen.

Ein weiterer Gegenstand der vorliegenden Erfindung ist ferner ein kontinuierliches Verfahren zur Herstellung von monomeren α-Cyanacrylsäureestern aus Cyanessigsäureestern und Formaldehyd oder Formaldehyd abspaltenden Verbindungen, dadurch gekennzeichnet, daß man in einer ersten Stufe Cyanessigsäureester und Formaldehyd bzw. Formaldehyd abspaltende Verbindungen in einer Zone kontinuerilicher Durchmischung mit basischen Katalysatoren versetzt, das Reaktionsgemisch unter fortgesetzter Durchmischung in einer Zone mit Temperaturen von 50°C bis 200°C kontinuierlich unter einem Druck von mehr als 1 bis 100 bar zur Reaktion bringt, nach einer Verweilzeit von maximal 20 Minuten kontinuierlich bei 50°C bis 200°C und einem Druck von 1 mbar bis 300 mbar kontinuierlich entgast, das entgaste Reaktionsgemisch in einer zweiten Stufe kontinuierlich mit Radikalinhibitoren, Anionenfängern und gegebenenfalls Wärmeträgern versetzt, das Gemisch durch eine Zone kontinuierlicher Entgasung und Durchmischung bei 150°C bis 320°C und 0,1 bis 50 mbar führt, die entstehenden Spaltrückstände kontinuierlich austrägt und die Entgasungsdämpfe kontinuierlich durch eine Zone mit einer Temperatur von 120°C bis 200°C führt in der höhersiedende Nebenprodukte kontinuierlich abgetrennt werden und in einer Zone bei einer Temperatur von -30°C und 50°C das entstandene α-Cyanacrylat kontinuierlich kondensiert.

Auch bei dieser Variante des erfindungsgemäßen Verfahrens werden Cyanessigester und Formaldehyd bzw. Formaldehyd abspaltende Verbindungen im Molverhältnis 1:2 bis 2:1, vorzugsweise 1:1,5 bis 1,5:1, besonders bevorzugt von 1:1,2 bis 1,2:1 eingesetzt.

Als Radikalinhibitoren können übliche Radikalfänger eingesetzt werden, z.B. Phenole wie Hydrochinon, p-Methoxyphenol, di-tert.-Butylphenol und tert.-Butylbrenzkatechin, vorzugsweise Hydrochinon.

Sie werden am besten in einer Menge von 1 Gew.-% bis 10 Gew.-% (bezogen auf das entgaste Reaktionsgemisch), bevorzugt in einer Menge von 2 Gew.-% bis - 8 Gew.-% (bezogen auf das entgaste Reaktionsgemisch) zugesetzt. Besonders bevorzugt wird ein Zusatz von 4 Gew.-% bis 6 Gew.-% Radikalinhibitor.

Unter Anionenfängern sind beispielsweise Phosphorverbindungen wie Phosphorpentoxid, Polyphosphorsäuren, Antimonverbindungen wie Antimonpentoxid, organische Säuren wie Pikrinsäure, Maleinsäureanhydrid, anorganische Verbindungen wie Eisen(III)-chlorid, $SO_2$ oder Fluorwasserstoff, bevorzugt substituierte oder unsubstituierte Aryl- oder Alkylsulfonsäuren wie Methansulfonsäure, Trifluormethansulfonsäure oder p-Toluolsulfonsäure oder Alkylsulfonsäureanhydride wie Methansulfonsäureanhydrid, Nonafluorbutansulfonsäureanhydrid oder p-Toluolsulfonsäureanhydrid genannt.

Wenn als Anionenfänger nicht verdampfbare Verbindungen wie Phosphorpentoxid, Polyphosphorsäure oder Antimonpentoxid eingesetzt werden, dann ist es vorteilhaft, zusätzlich einen unter Reaktionsbedingungen flüchtigen Anionenfänger wie $SO_2$, Methansulfonsäure oder Methansulfonsäureanhydrid einzusetzen.

Als günstig hat es sich erwiesen, 1 Gew.-% bis 10 Gew.-%, bevorzugt 2 Gew.-% bis 8 Gew.-%, besonders bevorzugt 4 Gew.-% bis 6 Gew.-% (bezogen auf das entgaste Reaktionsgemisch) eines nicht verdampfbaren Anionenfängers und gleichzeitig 10 ppm bis 10.000 ppm (bezogen auf das entgaste Reaktionsgemisch) an flüchtigen Anionenfängern zuzusetzen.

Hochsiedende organische Lösungsmittel wie Trikresylphosphat, Triphenylphosphat, Ethylenglykoldimethylether oder Triethylenglykoidimethylether können als Wärmeträger eingesetzt werden.

Das erfindungsgemäße Verfahren zur Herstellung von Poly-α-cyanacrylaten und deren Depolymerisation kann wie folgt durchgeführt werden (siehe Figur 2):

Paraformaldehyd und Cyanessigsäureester werden in dem Rührbehälter R1 eingewogen und in den Rührbehälter R2 überführt. Von dort wird der Extruder S1 kontinuierlich mit diesem Gemisch über die Pumpe P1 beschickt. In dem Behälter B1 wird der basische Katalysator oder dessen Lösung vorgelegt und mit der Pumpe P2 kontinuierlich in den Extruder gedrückt. Durch die Vakuumpumpe VI wird an einem Entgasungsturm des Extruders S1 Vakuum angelegt, so daß der basische Katalysator das Reaktionswasser und andere flüchtige Verbindungen verdampft und im Kühler W1 kondensiert werden. Dieses Destillat wird im Behälter B2 gesammelt und ausgetragen. Von der Düse des Extruders wird die Polycyanacrylatschmelze auf das Transportband S2 gebracht und dort vom Ventilator V4 gekühlt, so daß das polymere Zwischenprodukt von der Mühle Z1 zerkleinert und in den Vorlagebehälter B3 der Dosierwaage S3 gebracht werden kann. Von dort wird das Polycyanacrylat kontinuierlich in den Extruder S6 geführt und kontinuierlich mit Hydrochinon und Phosphorpentoxid aus den Behältern B4 und B5 über die Dosierwaagen S4 und S5 versetzt. In den Extruder S6 wird aus dem Behälter R3 über die Pumpe PJ $SO_2$ oder Methansulfonsäure evtl. gelöst in Trikresylphosphat gepumpt. Durch die Vakuumpumpe V2 wird an einem Entgasungsturm des Extruders S6 Vakuum angelegt, die entstehenden flüchtigen Reaktionsprodukte in den Abscheider F1 geleitet der bei einer Temperatur von 120°C bis 200°C gehalten wird. Dadurch werden hochsiedende Reaktionsnebenprodukte kondensiert und durch die Pumpe P4 ausgetragen, während die verbleibenden Dämpfe in dem Kühler W2 kondensiert und entweder direkt in den Rührbehälter R5 geleitet werden, oder direkt zur Feindestillation in den Dünnschichtverdampfer K2 geleitet werden. Die nicht verdampfbaren Rückstände werden durch die Düse von S6 ausgetragen. Aus K2 wird das Sumpfprodukt durch die Pumpe P5 ausgetragen und verworfen, während der gereinigte Cyanacrylatdampf im Vakuum (durch die Vakuumpumpe V3) in den Kühler W3 geführt, dort kondensiert und in den Rührbehälter R5 geführt wird. Im Rührbehälter R5 können noch Stabilisatoren gegen radikalische und anionische Polymerisation zugesetzt werden, bevor das Cyanacrylat über den Behälter B 11 ausgetragen wird.

Der große Vorteil des erfindungsgemäßen Verfahrens zur Herstellung von Poly-α-cyanacrylsäureestern und deren Depolymerisation besteht darin, daß erstmals, ausgehend von Cyanessigsäureestern und Formaldehyd

oder Formaldehyd abspaltenden Verbindungen, in voll kontinuierlicher Fahrweise monomere α-Cyanacrylsäureester hergestellt werden können, ohne daß aufwendige und zeitintensive Trocknungs- oder Reinigungsverfahren in Kauf genommen werden müssen. Man gelangt direkt von den monomeren Ausgangsprodukten zum monomeren Endprodukt, welches als hochwertiger Klebstoff verwendet werden kann. Die Ausbeute an monomeren α-Cyanacrylat kann dabei mehr als 70 % (bezogen auf Cyanessigsäureester) betragen.

Die Produkte sind wochen- und monatelang lagerstabil und können nach bekannten Verfahren weiter verarbeitet werden.

**Beispiel 1**

Eine Zweiwellenschneckenpresse (siehe Fig. 1) mit einem Wellendurchmesser von 32 mm und einer Länge von 1250 mm wird über eine Pumpe mit 10 kg/h eines Gemisches aus 76,6 Gew.-% Cyanessigsäuremethylester und 23,4 Gew.-Paraformaldehyd kontinuierlich beschickt. In der Aufheizzone (2) wird das Gemisch auf 100°C erwärmt und in der Dosierzone (3) kontinuierlich mit 480 ml/h einer 20 %igen Lösung von Piperidin in Ethylenglykoldimethylether versetzt. Das Reaktionsgemisch wird bei 100°C bis zur Entgasungszone gefördert und dort bei 60 mbar kontinuierlich entgast. Pro Stunde werden 1900 ml an flüchtigen Reaktionsprodukten abdestilliert. An der Düse des Extruders werden pro Stunde 8,5 kg Poly-α-cyanacrylsäuremethylester als hellgelbe Schmelze isoliert.

Das Produkt besitzt ein durchschnittliches Molekulargewicht von 1065 (gemessen durch Dampfdruckosmometrie in Aceton).

**Spaltung des Polycyanacrylates:**

700 g Polycyanacrylat hergestellt nach Beispiel 1 werden mit 30 g Phosphorpentoxid und 30 g Hydrochinon versetzt, unter Rühren und Einleiten von $SO_2$ evakuiert und erhitzt. Bei 155°C Sumpftemperatur und 2 mbar Druck wird eine Destillationsbrücke destilliert und die Sumpftemperatur auf 200°C gesteigert. Es werden dabei 580 g α-Cyanacrylsäuremethylester-Rohware in einer auf -70°C gekühlten Vorlage gesammelt.

Dieses Reaktionsprodukt eignet sich vorzüglich zur Formulierung von hochwertigen Cyanacrylat-Klebstoffen.

**Beispiel 2**

Ein Extruder wie in Beispiel 1, jedoch mit einer Länge von 1450 mm und zwei hintereinander liegenden Polymerisations- und Entgasungszonen wird mit 3 kg/h einer Mischung aus 135 Gew.-Teilen Paraformaldehyd und 560 Gew.-Teilen Cyanessigsäureethylester beschickt. In der Zone 3 werden pro Stunde 120 g Piperidin eindosiert. Die Reaktionstemperatur wird innerhalb des Extruders gleichmäßig von 60°C auf 200°C (von Dosierzone bis zur Extrudierungszone) gesteigert. In der ersten Entgasungszone wird ein Vakuum von 800 mbar, in der zweiten 150 mbar angelegt, wobei die flüchtigen Reaktionskomponenten abdestilliert werden. In der Extrusionszone fallen pro Stunde 2400 g einer gelben Schmelze von Poly-α-cyanacrylsäureethylester an.

Das Polymerisat besitzt ein durchschnittliches Molekulargewicht von 820 (gemessen durch Dampfdruckosmometrie in Aceton).

**Beispiel 3**

Ein Extruder wie im Beispiel 2 wird mit 7 kg/h einer Mischung aus 30 Gew.-Teilen Formaldehyd und 147 Gew.-Teilen Cyanessigsäuremethylester beschickt. Die Temperatur wird bis zur Dosierzone (3) auf 140°C gesteigert. In Zone 3 werden pro Stunde 80 g Triethylamin eindosiert. Das Reaktionsgemisch wird in den weiteren Zonen auf 140°C gehalten. In der ersten Entgasungszone werden bei 100 mbar und in der zweiten Entgasungszone bei 30 mbar die flüchtigen Reaktionskomponenten abgezogen. In der Extrusionszone werden pro Stunde 4,3 kg Polycyanacrylsäuremethylester mit einem durchschnittlichen Molekulargewicht von 608 (gemessen in Aceton durch dampdruckosmometrie) erhalten.

**Beispiel 4**

Eine Apparatur wie aus Bild 2 ersichtlich wird aufgebaut, wobei die Reaktionsschnecken S1 und S6 Extruder vom Typ ZSK mit einem Wellendurchmesser von 32 mm und einem Verhältnis von Länge: Durchmesser von L:D = 40 besitzen. Der Entgasungsturm von S1 besitzt eine Länge von 200 mm, der von S6 eine Länge von 400 mm. Die Schnecke S1 wird durch Beheizung bei 120°C gehalten, während bei der Schnecke S6 die Temperatur von der Einzugszone bis zum Entgasungsturm stetig ansteigt, bis zur Temperatur des Entgasungsturmes von 300°C. Der Abscheider F1 wird durch Beheizung bei 185°C gehalten. Der Dünnschichtverdampfer K2 wird durch Beheizung bei 120°C gehalten. Die Wellendrehzahl von S1 beträgt 120 Upm, die von S6 beträgt 50 Upm. Der Druck am Entgasungsturm von S1 beträgt 100 mbar, an jenem von S6 beträgt 8 mbar. Der Dünnschichtverdampfer wird bei einem Druck von 1 mbar betrieben.

Alle Kühler werden mit Kaltwasser betrieben.

Im Rührbehälter R1 wird ein Gemisch aus Cyanessigsäuremethylester und Paraformaldehyd im Molverhältnis 1:1,1 vorgelegt und in den Rührbehälter R2 gedrückt. Von dort werden von dieser Suspension pro Stunde 11,8 kg mit der Pumpe P1 in die geschlossene Einzugszone des Extruders S1 gedrückt. Gleichzeitig werden aus dem Behälter B1 durch die Pumpe P2 pro Stunde 0,36 kg einer 50%igen Lösung von Piperidin in Ethylenglykoldimethylether in den Extruder S1 gepumpt. Nach der Reaktion im Extruder werden das Reaktionswasser, Piperidin, Ethylenglykoldimethylether und andere flüchtige Reaktionsprodukte im Entgasungsturm abdestilliert, im Kühler W1 kondensiert und über den Behälter B2 ausgetragen. Den Extruder S1 verläßt an der Düse eine hellgelbe Polymerschmelze, die durch den Ventilator V4 gekühlt, im Zerkleinerer Z1 gemahlen und über den Behälter B3 und die Dosierwaage S3 dem Extruder S6 zugeführt wird.

Der Extruder S6 wird gleichzeitig über die Behälter B4 und B5 und die Dosierwaagen S4 und S5 pro Stunde mit 0,5 kg Hydrochinon und 0,5 kg Phosphorpentoxid beschickt. Dieses Gemisch wird im Extruder S6 aus dem Behälter R3 mit der Pumpe P3 durch eine Düse mit 0,54 kg pro Stunde einer Lösung von 350 g/l $SO_2$ in Trikresylphosphat versetzt.

Aus dem Extruder S6 tritt aus der Düse ein schwarzer, hochviskoser Rückstand aus, der verworfen wird. Aus dem Entgasungsturm von S6 werden die verunreinigten Cyanacrylatdämpfe in den gerührten Abscheider F1 geführt, aus dem die kondensierten hochsiedenden Spaltnebenprodukte durch die Pumpe P4 ausgetragen werden. Das gereinigte Cyanacrylat wird im Kühler W2 kondensiert und durch das Reduzierventil V5 (V4 ist geschlossen) in den Dünnschichtverdampfer K2 geleitet. Dort wird das Sumpfprodukt durch die Pumpe P5 ausgetragen, während der hoch gereinigte Cyanacrylatdampf im Kühler W3 kondensiert und im Rührbehälter R5 gesammelt wird. Pro Stunde fallen im Behälter R5 7,1 kg an hochreinem α -Cyanacrylsäuremethylester an, die durch den Behälter BII ausgetragen werden.

**Beispiel 5**

Der Versuch aus Beispiel 4 wird wiederholt, wobei statt Cyanessigsäuremethylester ein Gemisch aus 35 % Cyanessigsäureallylester und 65 % Cyanessigsäuremethylester verwendet wird. Es werden pro Stunde 7 kg eines Gemisches aus 34 % Cyanacrylsäureallylester und 66 % Cyanacrylsäuremethylester erhalten.

**Erläuterung zu Fig. 2**

1... Zugabe von Cyanessigsäureestern
2... Zugabe von Paraformaldehyd
3... Rührbehälter R1
4... Rührbehälter R2
5... Dosierpumpe P1
6... Extruder S1
7... Zugabe von Piperidin
8... Behälter B1
9... Dosierpumpe P2
10.. Kühler W1
11.. Vakuumpumpe V1
12.. Abluft
13.. Behälter B2
14.. Abwasser
15.. Förderband S2
16.. Ventilator V4
17.. Zerkleinerer Z1
18.. Behälter B3
19.. Dosierwaage S3

20.. Zuführung
21.. Extruder S6
22.. Zugabe von Hydrochinon
23.. Behälter B4
24.. Dosierwaage S4
25.. Zugabe von $P_2O_5$
26.. Behälter B5
27.. Dosierwaage S5
28.. Zugabe des Wärmeträgers (Trikresylphosphat)
29.. Zugabe des Anionenfängers ($SO_2$)
30.. Behälter R3
31.. Dosierpumpe P3
32.. Spaltdampfleitung
33.. Abscheider F1
34.. Pumpe P4
35.. Abfall
36.. fester Rückstand
37.. Kühler W2
38.. Vakuumpumpe V2
39.. Abluft
40.. Ventil V5
41.. Dünnschichtverdampfer K2
42.. Pumpe P5
43.. Abfall
44.. Ventil V4
45.. Rührbehälter R5
46.. Dosierung von Stabilisatoren
47.. Behälter B11
48.. Cyanacrylat
49.. Kühler W3
50.. Vakuumpumpe V3
51.. Abluft

**Patentansprüche**

1. Verfahren zur Herstellung von Poly-α-cyanacrylsäureestern durch Umsetzung von Formaldehyd oder Formaldehyd abspaltenden Verbindungen mit Cyanessigsäureestern der Formel

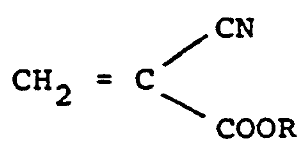

in welcher
R einen Alkyl-, Halogenalkyl-, Alkoxyalkyl, Cycloalkyl-, Alkenyl- oder Phenylrest mit jeweils 1-10 C-Atomen bedeutet, in Gegenwart 0,001 bis 0,05 Mol basischer Katalysatoren, dadurch gekennzeichnet, daß die Umsetzung kontinuierlich bei Temperaturen von 50 - 200°C, bei einem Druck von 1 bis 100bar und einer Verweilzeit von weniger als 20 Minuten durchgeführt wird, indem das Reaktionsgemisch in einen Reaktor kontinuierlich eingespeist, mit einem Katalysator vermischt, das Gemisch bei Reaktionstemperatur durch eine Reaktionszone geführt und flüchtige Substanzen abdestilliert werden.

2. Verfahren nach Anspruch 1 dadurch gekennzeichnet, daß das Molverhältnis von Formaldehyd zur Cyanessigsäureester 1 : 2 bis 2 : 1 beträgt.

3. Verfahren zur Herstellung von poly- α -cyanacrylsäureestern nach Anspruch 1, dadurch gekennzeichnet, daß das Reaktionsgemisch mehrere Reaktions- und Entgasungsstufen durchläuft.

4. Verfahren zur Herstellung von Poly-α-cyanacrylsäureestern nach Anspruch 1, dadurch gekennzeichnet,

daß der Katalysator in Form verdünnter Lösungen eingesetzt wird.

5. Verfahren zur Herstellung von Poly-α-cyanacrylsäureestern nach Anspruch 1, dadurch gekennzeichnet, daß Katalysatoren verwendet werden, die unter den Entgasungsbedingungen flüchtig sind und in den Entgasungszonen weitgehend destillativ abgetrennt werden.

6. Verwendung der gemäß Anspruch 1 hergestellten Polycyanacrylsäureester zur Herstellung von Monomeren α-Cyanacrylsäureestern.

7. Verwendung der gemäß Anspruch 1 hergestellten Polyα-cyanacrylsäureester zur Depolymerisation in der Schmelze in einer Reaktionsschnecke.

8. Kontinuierliches Verfahren zur Herstellung von α-Cyanacrylsäureestern der Formel 1

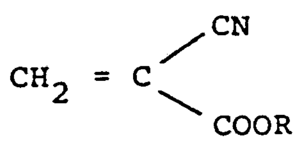

(I),

in welcher

R ein gesättigter oder ungesättigter Alkyl-, Cycloalkyl- Aralkyl- oder Alkoxyalkylrest mit 1 bis 10 C-Atomen ist,

dadurch gekennzeichnet, daß man in einer ersten Stufe Cyanessigsäureester und Formaldehyd bzw. Formaldehyd abspaltende Verbindungen in einer Zone kontinuierlicher Durchmischung mit basischen Katalysatoren versetzt, das Reaktionsgemisch unter fortgesetzter Durchmischung in einer Zone mit Temperaturen von 50° C bis 200° C kontinuierlich unter einem Druck von 1 bis 100 bar zur Reaktion bringt, nach einer mittleren Verweilzeit von maximal 20 Minuten kontinuierlich bei 50° C bis 200° C und einem Druck von 1 mbar bis 300 mbar und einer Verweilzeit von weniger als 10 Minuten kontinuierlich entgast, das entgaste Reaktionsgemisch in einer zweiten Stufe kontinuierlich mit Radikalinhibitoren, Anionenfängern und gegebenenfalls Wärmeträgern versetzt, das Gemisch durch eine Zone kontinuierlicher Entgasung und Durchmischung bei 150° C bis 320° C und 0,1 bis 50 mbar führt, die entstehenden Spaltrückstände kontinuierlich austrägt und die Entgasungsdämpfe kontinuierlich durch eine Zone mit einer Temperatur von 120° C bis 200° C führt, in der höhersiedende Nebenprodukte kontinuierlich abgetrennt werden, und in einer Zone bei einer Temperatur von -30° C bis 50° C das entstandene α-Cyanacrylat kontinuierlich kondensiert.

9. Verfahren gemäß Anspruch 8, dadurch gekennzeichnet, daß Cyanessigester und Formaldehyd bzw. Formaldehyd abspaltende Verbindungen im Molverhältnis 1:2 bis 2:1 eingesetzt werden.

10. Verfahren gemäß Anspruch 8, dadurch gekennzeichnet, daß die Radikalinhibitoren und Anionenfänger in einer Menge von jeweils 1 Gew.-% bis 10 Gew.-%, bezogen auf eingesetzten Cyanessigsäureester kontinuierlich zugegeben werden.

11. Verfahren gemäß Anspruch 8, dadurch gekennzeichnet, daß mindestens ein Anionenfänger zugesetzt wird, der unter den angegebenen Reaktionsbedingungen flüchtig ist.

12. Verfahren gemäß Anspruch 8, dadurch gekennzeichnet, daß als Reaktor in der ersten und zweiten Stufe je ein Apparat vom Typ eines Extruders verwendet wird.

## Claims

1. Process for the production of poly-α-cyancacrylic acid esters by reacting formaledehyde or compunds releasing formaldehyde with cyanoacetic acid esters of the formula

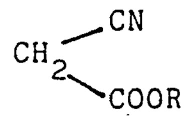

in which
R denotes an alkyl, halogenoalkyl, alkoxyalkyl, cycloalkyl, alkenyl or phenyl radical, each having 1-10 C atoms,

in the presence of 0.001 to 0.05 mol of basic catalysts, characterized in that the reaction is carried out continuously at temperatures of 50 - 200° C, under a pressure of 1 to 100 bars and with a residence time of less than 20 minutes by continuously introducing the reaction mixture into a reactor, mixing it with a catalyst, passing the mixture through a reaction zone at the reaction temperature and distilling off volatile substances.

2. Process according to Claim 1, characterized in that the molar ratio of formaldehyde to cyanoacetic acid ester is 1:2 to 2:1.

3. Process for the production of poly-α-cyanoacrylic acid esters according to Claim 1, characterized in that the reaction mixture passes through several reaction and degassing zones.

4. Process for the production of poly-α-cyanoacrylic acid esters according to Claim 1, characterized in that the catalyst is used in the form of dilute solutions.

5. Process for the production of poly-α-cyanoacrylic acid esters according to Claim 1, characterised in that the catalysts used are volatile under the degassing conditions and are largely removed by distillation in the degassing zones.

6. Use of the polycyanoacrylic acid esters produced according to Claim 1 for the production of monomers α-cyanoacrylic acid esters.

7. Use of the poly-α-cyanoacrylic acid esters produced according to Claim 1 for depolymerization in the melt in a reaction screw.

8. Continuous process for the production of α-cyanoacrylic acid esters of the formula 1

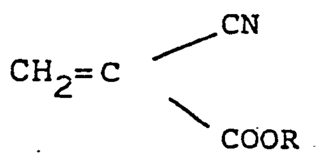 (I)

in which
R is a saturated or unsaturated alkyl, cycloalkyl, aralkyl or alkoxyalkyl radical with 1 - 10 C-atoms, characterized in that in a first stage cyanoacetic acid ester and formaldehyde or compounds releasing formaldehyde are mixed with basic catalysts in a zone of continuous thorough mixing, the reaction mixture is continuously reacted with continued thorough mixing in a zone having temperatures of 50° C to 200° C under a pressure of 1 to 100 bars, and after an average residence time of not more than 20 minutes the mixture is continuously degassed for a residence time of less than 10 minutes at 50° C to 200° C and a pressure of 1 mbar to 300 mbars, in a second stage radical inhibitors, anion traps and, optionally, heat carriers are continuously added to the degassed mixture, the mixture is passed through a zone of continuous degassing and thorough mixing at 150° C to 320° C and 0.1 to 50 mbars, the cleavage residues which form are continuously discharged and the degassing vapours are continuously passed through a zone having a temperature of 120° C to 200° C, in which higher-boiling by-products are continuously separated off and the α-cyanoacrylate produced is continuously condensed in a zone at a temperature of -30° C to 50° C.

9. Process according to Claim 8 characterized in that cyanoacetic esters and formaldehyde or compounds releasing formaldehyde are used in a molar ratio of 1:2 to 2:1.

10. Process according to Claim 8, characterized in that the radical inhibitors and anion traps are added

continuously in a quantity of in each case 1 % by weight to 10 % by weight, based on the cyanoacetic acid ester employed.

11. Process according to Claim 8, characterized in that at least one anion trap is added which is volatile under the indicated reaction conditions.

12. Process according to Claim 8, characterized in that the reactors used in the first and second stages are each an apparatus of the extruder type.

**Revendications**

1. Procédé de fabrication de polyesters α -cyanacryliques par réaction de la formaldéhyde ou de composés libérant de la formaldéhyde avec des esters cyanacétiques de formule :

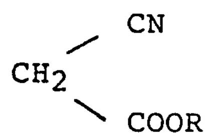

dans laquelle

R signifie un radical alcoyle, halogénoalcoyle, alcoxyalcoyle, cycloalcoyle, alcényle ou phényle ayant chacun 1 à 10 atomes de carbone,

en présence de 0,001 à 0,05 mole de catalyseurs basiques, caractérisé en ce qu'on exécute la réaction en continu à des températures de 50 à 200°C, sous une pression de 1 à 100 bars et avec un temps de séjour inférieur à 20 minutes, on alimente le mélange de réaction dans un réacteur en continu, on le mélange avec un catalyseur, on fait passer le mélange à la température de réaction à travers une zone de réaction et l'on sépare par distillation les substances volatiles.

2. Procédé selon la revendication 1, caractérisé en ce que le rapport molaire formaldéhyde : ester cyanacétique est de 1 : 2 à 2 : 1.

3. Procédé de fabrication de polyesters α-cyanacryliques selon la revendication 1, caractérisé en ce que le mélange de réaction parcourt plusieurs stades de réaction et de dégazage.

4. Procédé de fabrication de polyesters α-cyanacryliques selon la revendication 1, caractérisé en ce qu'on utilise le catalyseur sous la forme de solutions diluées.

5. Procédé de fabrication de polyesters α -cyanacryliques selon la revendication 1, caractérisé en ce qu'on utilise des catalyseurs qui dans les conditions du dégazage sont volatils et qui sont séparés largement par distillation dans les zones de dégazage.

6. Utilisation des polyesters cyanacryliques préparés selon la revendication 1 pour la fabrication d'esters α -cyanacryliques monomères.

7. Utilisation des polyesters α-cyanacryliques préparés selon la revendication 1 pour la dépolymérisation en masse fondue dans une vis sans fin de réaction.

8. Procédé continu de fabrication d'esters α -cyanacryliques de formule I :

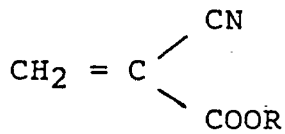

(I)

dans laquelle

R est un radical alcoyle, cycloalcoyle, aralcoyle ou alcoxyalcoyle saturé ou non saturé ayant 1 à 10 atomes de carbone,

caractérisé en ce que dans un premier stade on ajoute à l'ester cyanacétique et à la formaldéhyde ou aux composés libérant de la formaldéhyde, dans une zone de mélange intime continu, des catalyseurs basiques, on fait réagir le mélange de réaction tout en continuant à mélanger intimement dans une zone à des températures de 50°C à 200°C de manière continue sous une pression de 1 à 100 bars, on dégaze après un temps de séjour moyen au maximum de 20 minutes continuellement à 50°C à 200°C sous une pression de 1 mbar à 300 mbars et pendant un temps de séjour inférieur à 10 minutes de manière continue, on ajoute au mélange de réaction dégazé dans un second stade continuellement des inhibiteurs de radicaux, des capteurs d'anions et éventuellement des caloporteurs, on fait passer le mélange à travers une zone de dégazage continu et de mélange intime à 150°C à 320°C sous 0,1 à 50 mbars, on élimine les résidus de clivage obtenus continuellement et l'on fait passer continuellement les vapeurs de dégazage à travers une zone à une température de 120°C à 200°C, dans laquelle on sépare continuellement les sous-produits à point d'ébullition élevé et l'on condense continuellement l' α-cyanacrylate obtenu dans une zone à une température de -30°C à 50°C.

9. Procédé selon la revendication 8, caractérisé en ce qu'on utilise l'ester cyanacétique et la formaldéhyde ou les composés libérant de la formaldéhyde dans le rapport molaire de 1:2 à 2:1.

10. Procédé selon la revendication 8, caractérisé en ce qu'on ajoute en continu les inhibiteurs de radicaux et les capteurs d'anions chacun en une quantité de 1% en poids à 10% en poids par rapport à l'ester cyanacétique utilisé.

11. Procédé selon la revendication 8, caractérisé en ce qu'on ajoute au moins un capteur d'anions qui est volatil dans les conditions de réaction indiquées.

12. Procédé selon la revendication 8, caractérisé en ce qu'on utilise comme réacteur dans le premier et le second stade chaque fois un appareil du type d'une extrudeuse.

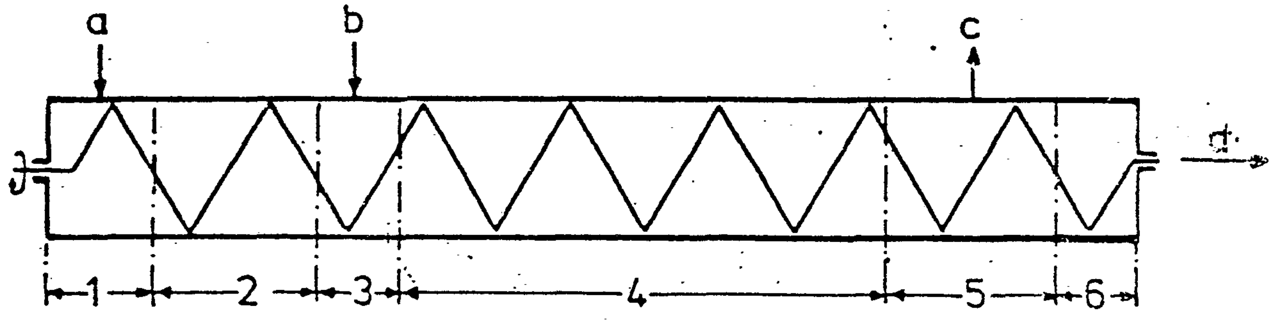


FIG. 1

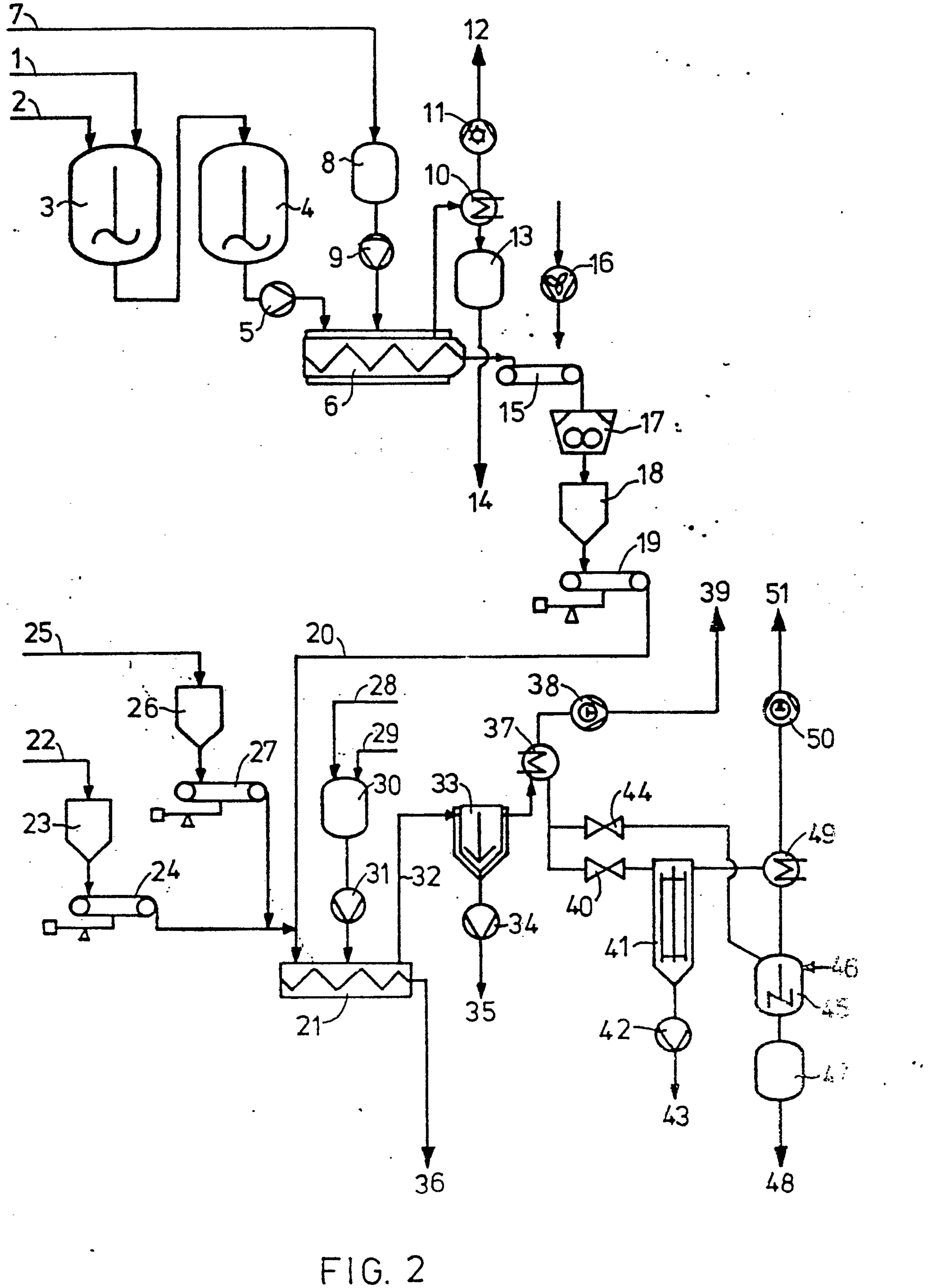
7
1
2
12
11
10
8
9
3
4
5
6
13
16
15
17
14
18
19
39
51
25
20
38
50
26
28
37
27
29
22
30
33
44
23
24
31
32
49
40
41
46
34
45
21
35
42
47
43
36
48

FIG. 2